# EUROPÄISCHE PATENTANMELDUNG

(11) **EP 3 020 421 A1**
(43) Veröffentlichungstag der Anmeldung: **18.05.2016**
(21) Anmeldenummer: 15194553.2
(22) Anmeldetag: 13.11.2015
(51) Int. Cl.: A61L 2/18, C02F 1/00, C02F 1/68, F24D 17/00, C02F 1/50

(54) **VORRICHTUNG ZUR WARNUNG VOR VERKEIMUNG IN WASSERLEITUNGEN UND GERÄTEN**

(30) Priorität: 13.11.2014 DE 102014116577
(71) Anmelder: Schulz GmbH, 58540 Meinerzhagen (DE)
(72) Erfinder: Schulz, Hartmut, 58540 Meinerzhagen (DE)
(74) Vertreter: Patentanwälte Dörner & Kötter PartG mbB

(57) **Zusammenfassung**

Die Erfindung betrifft eine Vorrichtung zur Warnung vor Verkeimung in Wasserleitungen und Geräten, umfassend einen Wasser-Zulauf (1), der mit mindestens einer Entnahmestelle (7) in Verbindung steht, wobei zwischen Wasser-Zulauf (1) und Entnahmestelle (7) eine Impfstelle (18) angeordnet ist, an der eine Zuführung (19) für ein Desinfektionsmittel vorgesehen ist, welches mit Hilfe einer Dosierpumpe (9) der Impfstelle (18) zuführbar ist. Es ist ein Strömungswächter (8) vorgesehen, der mit einer Mess- und Steuereinrichtung (12) in Verbindung steht.

## Beschreibung

Die Erfindung betrifft eine Vorrichtung zur Warnung vor Verkeimung in Wasserleitungen und Geräten, umfassend einen Wasser-Zulauf, der mit mindestens einer Entnahmestelle in Verbindung steht, wobei zwischen Wasser-Zulauf und Entnahmestelle eine Impfstelle angeordnet ist, an der ein Zulauf für ein Desinfektionsmittel vorgesehen ist, welches mit Hilfe einer Dosierpumpe der Impfstelle zuführbar ist.

Innerhalb von Installationssystemen für Trinkwasser sind in der Regel eine Vielzahl von Entnahmestellen vorgesehen. Insbesondere bei öffentlichen Gebäuden, zum Beispiel Schulen, Badeanstalten oder Krankenhäusern, kann die Anzahl der Entnahmestellen sehr hoch sein. Zur Vermeidung von Keimbildung in Installationssystemen, beispielsweise durch Legionellen, sind verschiedene Maßnahmen bekannt. So ist aus der EP 1 652 822 A1 ein Verfahren zur Überwachung und Desinfektion von Verteilungssystemen für Warmwasser bekannt, bei dem in regelmäßigen Abständen die Konzentration des Desinfektionsmittels im Wasser, die Temperatur des Wassers im Vorratstank bzw. im Rücklauf gemessen werden. Aus der GB 2 348 945 A ist ein Warmwasser - Installationssystem mit einer Vielzahl von Entnahmestellen bekannt, bei dem Wasser in einem Niedrigtemperaturbereich kontinuierlich umgewälzt wird und dem Wasser ein Desinfektionsmittel beigemischt wird. Auf Anforderung wird heißes Wasser aus einem Vorratstank entnommen und mit niedrig temperiertem und mit Desinfektionsmittel versehenem Wasser gemischt.

Innerhalb der Installationssysteme sind die Entnahmestellen jedoch unterschiedlich häufig frequentiert. Während beispielsweise in Krankenhäusern Entnahmestellen in Küchen, Waschräumen und dergleichen häufig benutzt werden, können in anderen Bereichen die Entnahmestellen deutlich weniger benutzt werden, beispielsweise in der Pathologie oder der Bäderabteilung. Aufgrund der wesentlich geringeren Anzahl an Betätigungen an einzelnen Entnahmestellen befindet sich in den Zuleitungen zu diesen Entnahmestellen Wasser, das zwischen den jeweiligen Entnahmen über einen längeren Zeitraum ruht, sogenanntes "Stagnationswasser". Dieses Wasser neigt zur Keimbildung, was im Falle einer Entnahme zu gesundheitlichen Beeinträchtigungen führen kann. Das gilt auch für die an die Wasserleitungen angeschlossenen Geräte, die bei Nicht-Gebrauch durch das verkeimte Stagnationswasser belastet werden. Insbesondere im Krankenhaus- und Pflegebereich gilt es aufgrund der sehr hohen Anforderungen, die sich aus den jeweiligen Hygienevorschriften ergeben, die Entnahme von mit Keimen belastetem Wasser zu vermeiden. Typische Geräte, für die die vorstehende Problematik zutrifft, sind zum Beispiel Dosiergeräte für die Bereitstellung von Desinfektionsgebrauchsmittellösungen, wie sie zum Beispiel aus der DE 103 42 952 A1 bekannt sind.

Die Vermeidung von Keimbildung in den Leitungen und / oder Geräten ist jedoch aufgrund der unregelmäßigen Entnahmen von Wasser bzw. Desinfektionsgebrauchsmittellösungen problematisch. In der Regel liegen keine Informationen vor, wann das letzte Mal an der jeweiligen Entnahmestelle Wasser entnommen wurde, was unter anderem eine Folge der manuellen Betätigung der Geräte ist.

Weil bei den bekannten Vorrichtungen die jeweilige Bedienperson keine Angabe dazu hat, wann das letzte Mal an der jeweiligen Entnahmestelle Wasser entnommen wurde, wird entweder Wasser oder Desinfektionsmittel durch die Leitungen und Geräte gespült, obwohl dies noch gar nicht notwendig ist oder die jeweilige Bedienperson versäumt es, die Leitungen mit Desinfektionsmitteln zu durchspülen, weil sie keine Kenntnis davon hat, wie lange die jeweilige letzte Entnahme zurückliegt. Hieraus ergibt sich einerseits das Problem, dass aufgrund der vorsorglichen häufigen Desinfektion ein sehr hoher Verbrauch an Desinfektionsmitteln auftritt. Andererseits kann es zu Wasserentnahmen kommen, die keimbelastet sind, wenn trotz lange zurück liegender Entnahme, also einer langen Stagnation des Wassers in der Leitung bzw. in dem Gerät, keine Spülung erfolgt.

Hier will die Erfindung Abhilfe schaffen. Der Erfindung liegt die Aufgabe zu Grunde, eine Vorrichtung zur Warnung vor Verkeimung in Wasserleitungen und Geräten zu schaffen, die eine bedarfsgerechte Spülung der Leitungen und Geräte ermöglicht, um die Entnahme von durch Stagnation verkeimtem Wasser zu vermeiden. Gemäß der Erfindung wird diese Aufgabe durch die Merkmale des Patentanspruchs 1 gelöst.

Mit der Erfindung ist eine Vorrichtung zur Warnung vor Verkeimung in Wasserleitungen und Geräten durch lange andauernde Stagnation geschaffen, die eine bedarfsgerechte Spülung der Wasserleitungen und Geräte ermöglicht. Dies ist dadurch hervorgerufen, dass ein Strömungswächter vorgesehen ist, der die Entnahme von Wasser in den Leitungen und Geräten überwacht und Signale an eine Mess- und Steuereinrichtung weiterleitet. Die Mess- und Steuereinrichtung misst die Dauer zwischen zwei Entnahmen und kann über voreingestellte Werte bedarfsgerecht das Bedienpersonal benachrichtigen. Hierdurch ist immer gewährleistet, dass im Falle einer lang andauernden Stagnation des Wassers in einer Leitung oder auch in einem Gerät das Bedienpersonal bei der nachfolgenden Entnahme Maßnahmen ergreift, um die Benutzung von verkeimtem Wasser zu vermeiden.

In Weiterbildung der Erfindung ist eine Alarmierungseinrichtung vorgesehen. Die Alarmierung kann optisch oder akustisch erfolgen. Mit Hilfe der Alarmierungseinrichtung wird das Bedienpersonal auf die lang andauernde Stagnation des Wassers in der Leitung aufmerksam gemacht. Das Bedienpersonal ist dadurch zuverlässig in die Lage versetzt, die Desinfektion selbstständig auszulösen und zu kontrollieren, bevor eine Verkeimung auftritt.

Vorteilhaft beinhaltet die Messeinrichtung eine Zeitmessung. Die Zeitmessung bietet die Möglichkeit, die Vorgaben für eine Desinfektion im Falle einer Stagnation genau einzustellen. Diese kann dann unabhängig von der Menge des zuletzt entnommenen Wassers ausgelöst werden.

Andere Weiterbildungen und Ausgestaltungen der Erfindung sind in den übrigen Unteransprüchen angegeben. Ein Ausführungsbeispiel der Erfindung ist in den Zeichnungen dargestellt und wird nachfolgend im Einzelnen beschrieben. Es zeigen:

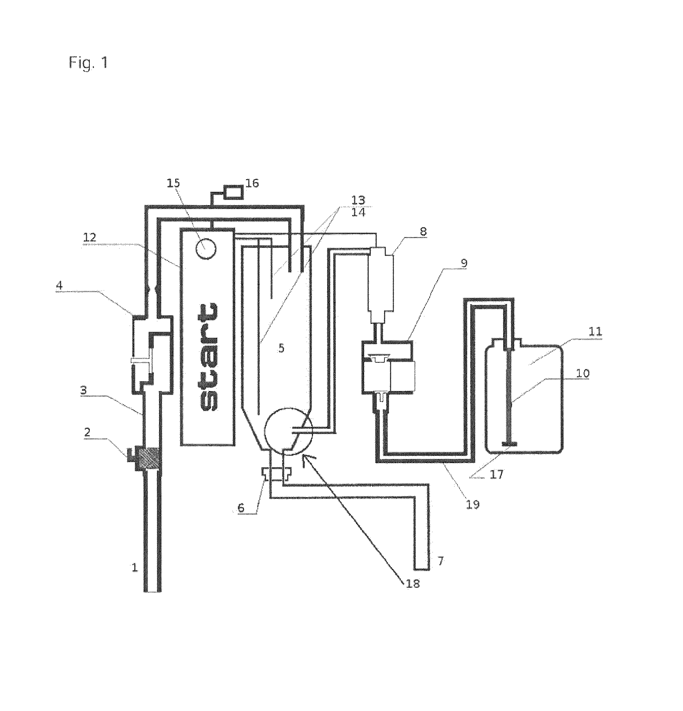

- Figur 1: eine Funktionsgruppendarstellung der erfindungsgemäßen Vorrichtung.

Die Vorrichtung zur Warnung vor Verkeimung in Wasserleitungen und / oder Geräten nach der Erfindung ist für den Einsatz im Krankenhaus- und Pflegebereich bestimmt. Im Ausführungsbeispiel ist in ein Dosiergerät für die Bereitstellung einer Desinfektionsgebrauchsmittellösung integriert. Sie besteht im Wesentlichen aus (Rohr-)Leitungen, und Komponenten, die mittels der Leitungen miteinander in Verbindung stehen. Im Ausführungsbeispiel ist ein Wasserzulauf 1 an ein Installationssystem für Trinkwasser angeschlossen. Der Wasserzulauf 1 ist über einen Schmutzfänger 2, einen Mengenregler 3 und ein Magnetventil 4 in eine Systemtrennung 5 geführt. In die Systemtrennung 5 fällt das einlaufende Wasser über eine freie Strecke, so dass Einlauf und Auslauf getrennt sind. Bei Nicht-Benutzung der Vorrichtung ist die Systemtrennung 5 vollständig entleert. Dasselbe gilt nach Beendigung eines Zapfvorgangs.

Die Systemtrennung 5 enthält Niveau-Sensoren 13, 14, von denen der Sensor 13 für die Ermittlung des Höchstfüllstands, der Sensor 14 für die Ermittlung des Mindestfüllstands verantwortlich ist. Eine Ausflussblende 6 ermöglicht den Ausfluss des Wassers über eine Entnahmestelle 7, z.B. einen Schwenkauslauf.

Die Sensoren 13, 14 sind an eine Mess- und Steuereinrichtung 12 angeschlossen. Die Füllstandsmessung erfolgt über die Mess- und Steuereinrichtung 12 in Verbindung mit den Niveau-Sensoren 13, 14 elektronisch. Bei Erreichen des Höchstfüllstandes spricht der Sensor 13 an und der Wasserzulauf 1 sperrt. Nach Ablauf einer eingestellten Zeit bzw. bei Erreichen des Mindestfüllstandes spricht der Sensor 14 an und der Wasserzulauf 1 wird vor Entleerung der Systemtrennung 5 wieder freigegeben, so dass in dem Wasservorlaufbehälter 5 immer eine ausreichende Wassermenge vorhanden ist. Die Entleerung der Systemtrennung 5 lässt sich bei Verwendung einer Ausflussdüse 6 mit konstantem Durchfluss zeitabhängig erfassen, wodurch der Wasserzulauf nach Verstreichen einer konstanten Zeit wieder freigegeben wird. Sollte einmal der Wasserzulauf unterbrochen sein, so erfolgt nach Ablauf einer Sperrzeit ein Alarm durch eine Signallampe 15.

In die Systemtrennung 5 mündet in einer Impfstelle 18 einer Zuführung 19 für Desinfektionsmittel. Die Zuführung 19 umfasst einen Strömungswächter 8 und eine Dosierpumpe 9. Die Dosierpumpe 9 ist frequenzgesteuert. Dies bietet im Verhältnis zum üblichen An- und Ausschalten der Pumpe den Vorteil, dass eine homogene Lösung kontinuierlich förderbar ist, da ständig eine gleich bleibende Menge Desinfektionsmittel gefördert wird. Die Zuführung 19 umfasst weiterhin eine Sauglanze 10. Die Sauglanze 10 reicht in einen Konzentratbehälter 11 mit Desinfektionsmittel. Die Sauglanze 10 ist an ihrem freien Ende mit einem Niveausensor 17 versehen, der den Mindestfüllstand im Konzentratbehälter 11 detektiert. Der Strömungswächter 8 ist ebenfalls mit einer Signallampe 15 verbunden.

Die Mess- und Steuereinrichtung 12 steht mit dem Strömungswächter 8 in Verbindung. Außerdem steht die Mess- und Steuereinrichtung 12 mit dem Mengenregler 3 in Verbindung. Sie ist folglich in der Lage, den Durchfluss des Wassers im Zulauf 1 als auch des Desinfektionsmittels in der Zuführung 19 zu messen.

Im Ausführungsbeispiel beinhaltet die Mess- und Steuereinrichtung 12 eine Zeitmessung. In Kombination mit der Verbindung mit dem Mengenregler 3 und dem Strömungswächter 8 kann die Vorrichtung daher feststellen, ob Wasser und / oder Desinfektionsmittel entnommen wurde bzw. welcher Zeitraum zwischen der letzten Entnahme von Wasser und / oder von Desinfektionsmittel vergangen ist. Darüber hinaus ist eine Alarmierungseinrichtung 16 vorgesehen. Die Alarmierungseinrichtung 16 ist mit der Mess- und Steuereinrichtung 12 verbunden. Sie kann beispielsweise auslösen, wenn der Zeitraum zwischen zwei Wasserentnahmen eine bestimmte Dauer überschreitet. Die Alarmierung weist den jeweiligen Bediener darauf hin, dass vor der nächsten Entnahme Maßnahmen zu ergreifen sind, um einer möglichen Verkeimung vorzubeugen. In der Regel ist eine optische Alarmierung vorgesehen. Selbstverständlich kann auch eine akustische Alarmierung vorgesehen sein.

Die erfindungsgemäße Vorrichtung ist manuell betätigbar. Hierbei werden im Falle einer Alarmierung von dem jeweiligen Bediener Maßnahmen ergriffen, die die Benutzung von verkeimtem Wasser verhindert. Die im Ausführungsbeispiel dargestellte Vorrichtung ist dabei derart aufgebaut, dass bei Auslösen der Beimengung von Desinfektionsmittel automatisch das Magnetventil 4 öffnet und Leitungswasser gefördert wird, so dass die Desinfektionsgebrauchsmittellösung am Auslauf 7 entnommen werden kann. Die Auslösung kann in einfacher Weise durch Öffnen der Zuführung 19 für das Desinfektionsmittel erfolgen, so dass dieses im Bereich der Impfstelle 18 dem Wasser zugeführt wird. Das Wasser-Desinfektionsgemisch strömt dann in Richtung des Auslaufs 7. Die Mess- und Steuereinrichtung 12 überwacht dabei die Menge an Wasser und / oder Desinfektionsmittel. Nach Durchlauf einer vorbestimmten Menge an Desinfektionsmittel, bspw. in der Größenordnung von 2,5 Litern, kann über die Alarmierungseinrichtung 16 ein Signal ausgesandt werden, die dem Bediener mitteilt, dass eine ausreichende Menge von Desinfektionsmittel und damit gleichzeitig auch eine ausreichende Menge an Wasser durchgelaufen ist und das möglicherweise mit Keimen belastete Stagnationswasser aus dem Wasserzulauf 1 abgelaufen ist. Die im Anschluss an diese Signalisierung entnommene Desinfektionsgebrauchsmittellösung ist aus Frischwasser hergestellt und daher nicht belastet, so dass der Gebrauch der Lösung unbedenklich ist.

Mit der Vorrichtung nach der vorliegenden Erfindung ist die Möglichkeit geschaffen, das Bedienpersonal rechtzeitig vor der Entstehung von Keimen in den Leitungen und Geräten bis zur Entnahmestelle zu warnen, in dem die Messund Steuereinrichtung 12, die vorzugsweise eine Zeitmessung beinhaltet, detektiert, in welchen Abständen Wasser entnommen wird. Bei Überschreiten eines bestimmten Zeitraums des Nichtgebrauchs erfolgt eine Alarmierung, die den jeweiligen Bediener wenn nicht unmittelbar so jedenfalls vor der nächsten Benutzung zur Ergreifung erforderlicher Maßnahmen auffordert.

Die erfindungsgemäße Vorrichtung ist dabei nicht zur Überwachung der Menge der Dosierung des Desinfektionsmittels vorgesehen, sondern sie dient der Überwachung und Warnung vor Stagnation in den Leitungen und Geräten. Auch erfolgt eine Überwachung, ob überhaupt eine Entnahme von Desinfektionsmittel erfolgt. Im Falle der Nicht-Benutzung - sei es durch einen technischen Defekt oder aufgrund eines manuellen Bedienfehlers - erfolgt ebenfalls eine Alarmierung. Die Beseitigung des kontaminierten Wassers erfolgt dann spätestens vor der nächsten Entnahme durch das Bedienpersonal durch manuelles Bedienen der Vorrichtung und damit im Gegensatz zu den automatisierten und kontinuierlich arbeitenden Systemen, die bspw. von Trinkwasserinstallationssystemen bekannt sind.

Im Ausführungsbeispiel ist die erfindungsgemäße Vorrichtung zur Warnung vor Verkeimung in Wasserleitungen und Geräten in ein Dosiergerät für die Bereitstellung einer Desinfektionsgebrauchsmittellösung integriert. Folglich ist die Benutzung von bereits vorhandenen Komponenten des Dosiergeräts möglich, was die Integration der erfindungsgemäßen Vorrichtung in die Geräte vereinfacht und außerdem die Kosten reduziert. Im Ausführungsbeispiel gilt dies bspw. für den Strömungswächter 8, die Mess- und Steuereinrichtung 12 sowie die Sensoren 13, 14.

## Patentansprüche

1. Vorrichtung zur Warnung vor Verkeimung in Wasserleitungen und Geräten, umfassend einen Wasser-Zulauf (1), der mit mindestens einer Entnahmestelle (7) in Verbindung steht, wobei zwischen Wasser-Zulauf (1) und Entnahmestelle (7) eine Impfstelle (18) angeordnet ist, an der eine Zuführung (19) für ein Desinfektionsmittel vorgesehen ist, welches mit Hilfe einer Dosierpumpe (9) der Impfstelle (18) zuführbar ist, **dadurch gekennzeichnet, dass** ein Strömungswächter (8) vorgesehen, der mit einer Mess- und Steuereinrichtung (12) in Verbindung steht.

2. Vorrichtung nach Anspruch 1, **dadurch gekennzeichnet, dass** eine Alarmierungseinrichtung (16) vorgesehen ist.

3. Vorrichtung nach Anspruch 1 oder 2, **dadurch gekennzeichnet, dass** die Mess- und Steuereinrichtung (12) eine Zeitmessung beinhaltet.

4. Vorrichtung nach einem oder mehreren der vorgenannten Ansprüche, **dadurch gekennzeichnet, dass** die Zuführung (19) für das Desinfektionsmittel manuell betätigbar ist.

5. Vorrichtung nach einem oder mehreren der vorgenannten Ansprüche, **dadurch gekennzeichnet, dass** die Zuführung (19) für das Desinfektionsmittel automatisch betätigbar ist.
